# FASCICULE DE BREVET EUROPEEN

(11) **EP 2 315 575 B1**
(45) Date de publication et mention de la délivrance du brevet: **05.04.2017**
(21) Numéro de dépôt: 09784482.3
(22) Date de dépôt: 07.07.2009
(51) Int. Cl.: A61K 8/27, A61Q 17/04, A61K 8/97, C04B 35/14, A61K 8/02, B82Y 5/00, C09C 1/00

(54) **COMPOSITION COSMÉTIQUE COMPRENANT UN MATÉRIAU COMPOSITE À BASE DE ZINC**
KOSMETISCHE ZUSAMMENSETZUNG ENTHALTEND ZINK-KOMPOSITMATERIALIEN
COSMETIC COMPOSITION COMPRISING A ZINC COMPOSITE MATERIAL

(30) Priorité: 07.07.2008 FR 0854602
(43) Date de publication de la demande: 04.05.2011
(73) Titulaire: Olmix, 56580 Brehan (FR); Centre National de la Recherche Scientifique (C.N.R.S.), 75016 Paris (FR); Université de Haute Alsace, 68093 Mulhouse Cedex (FR)
(72) Inventeur: BRENDLE, Jocelyne, F-68270 Wittenheim (FR); KNOERR, Raphaël, 68500 Guebwiller (FR); DEMAIS, Hervé, 56390 Brandivy (FR); PATARIN, Joël, Clovis, Emile, 68720 Flaxlanden (FR); LEBEAU, Bénédicte, Marie, Thérèse, 68700 Wattwiller (FR)
(74) Mandataire: Lavoix
(86) Numéro de dépôt international: PCT/FR2009/051348
(87) Numéro de publication internationale: WO 2010/004208

(56) Documents cités:
- FR-A1- 2 622 441
- FR-A1- 2 674 126
- GB-A- 960 639
- US-A1- 2003 099 763
- US-A1- 2007 128 707
- YE CAI, KENNETH H. SANDHAGE: "Zn2SiO4-coated microparticles with biologically-controlled 3D shapes" PHYSICA STATUS SOLIDI (A), vol. 202, no. 10, 2005, pages R105-R107, XP002499834
- ZHAOTING LIU ET AL: "Synthesis of ZnFe2O4/SiO2 composites derived from a diatomite template" BIOINSPIRATION & BIOMIMETICS, vol. 2, no. 1, 2007, pages 30-35, XP002499835

## Description

La présente invention a pour objet des compositions cosmétiques comprenant un matériau composite en association avec un véhicule cosmétiquement acceptable, ledit matériau composite comprenant de la terre de diatomée et une phase à base de zinc, dans laquelle la phase à base de zinc du matériau composite est une phase cristalline à base d'oxyde de zinc (II).

Les terres de diatomée sont des algues eucaryotes fossilisées microscopiques (∼1-200 µm) trouvant leurs origines principalement dans les zones lacustres. Elles sont protégées par une enveloppe dure appelée frustule, de composition chimique générale : 80% de silice, 5% d'alumine, 5% d'oxyde de calcium et le complément d'autres minéraux. Aussi, l'origine ainsi que l'âge du sédiment conditionnent les propriétés de ce matériau naturel. Par exemple, une terre de diatomée extraite de carrières près des mers, présente un taux de cristallinité de 60% et une composition en CaCO₃ de l'ordre de 25%, contre moins de 1% de cristallinité et de carbonate pour une terre de diatomée issue de gisement d'eau douce.

D'un point de vue structurel, la terre de diatomée présente des caractéristiques physiques intrinsèques remarquables de par la forte porosité de sa frustule par exemple (taille de pore unitaire du micromètre) constituant environ 40% du volume total. De fait, de nombreuses applications de ce matériau macroporeux sont possibles sans qu'aucune modification préliminaire ne soit requise. En effet, la porosité de la frustule peut être assimilée à un tamis moléculaire (J. Parkinson and R. Gordon, Nanotechnology, 1999, 17, 230), où la taille de pore est régie par la nature de l'espèce. Un exemple d'application de la terre de diatomée à l'état brut, est son utilisation en tant que media filtrant, en piégeant par interactions physiques des composés organiques de taille importante. De fait, il vient que la sélectivité des composés organiques à piéger est dépendante de la porosité de la terre de diatomée, et donc de la nature de la terre de diatomée considérée.

Il a été mis en évidence que la texture même de la frustule de la terre de diatomée est telle qu'elle est recouverte de pointes acérées, donnant lieu à des propriétés abrasives (Z. Korunic, J. stored Prod. Res., 1998, 34, 87-97; W. Quarles, IPM Practitioner, 1992, 14, 1-11 et W. Quarles, IPM Practitioner, 1996, 18, 1-10), largement mises en application au niveau industriel dans la lutte anti-parasitaire. D'autres applications de la terre de diatomée brute directement liées à ses propriétés physico-chimiques sont à relever. En effet, ce matériau est non inflammable, non soluble dans l'eau et extrêmement stable à l'air. Ainsi, la terre de diatomée trouve des applications dans l'isolation thermique (O. Unal, T. Uygunoglu and A. Yildiz, Building and Environment, 2007, 42, (2), 584), mais plus couramment en tant que charge fonctionnelle (X. Li, W. Chen, C. Bian, J. He, N. Xu and G. Xue, Applied Surface Science, 2003, 217, (1-4), 16) dans l'industrie de la peinture, du papier ou bien encore du caoutchouc.

La terre de diatomée est composée majoritairement de silice, présente soit sous forme de ponts siloxane (Si-O-Si) participant à la structure du squelette de la terre de diatomée, soit sous forme de groupements silanols (Si-OH) situés à la surface du matériau (P. Yuan, D. Q. Wu, H. P. He and Z. Y. Lin, Applied Surface Science, 2004, 227, (1-4), 30-39). Assimilés à des sites réactifs, ces derniers confèrent au matériau une réactivité chimique (J.-B. d'Espinose de la Caillerie, M. R. Aimeur, Y. E. Kortobi and A. P. Legrand, Journal of Colloid and Interface Science, 1997, 194, (2), 434-439) en conditionnant la charge, l'acidité, le caractère hydrophile de la surface, mais aussi les réactions d'échange de ligands et dite de «grafting» (greffage) permettant la fonctionnalisation de la terre de diatomée. Aussi, la modification de la terre de diatomée, à travers les silanols de surface, a pour objectif l'obtention de matériaux hybrides possédant des propriétés physico-chimiques nouvelles voire améliorées par rapport au matériau initial.

La présente invention est telle que définie dans l'objet des revendications 1 à 5 et concerne donc :
**1.** Composition cosmétique comprenant un matériau composite en association avec un véhicule cosmétiquement acceptable, ledit matériau composite comprenant de la terre de diatomée et une phase à base de zinc, dans laquelle la phase à base de zinc du matériau composite est une phase cristalline à base d'oxyde de zinc (II).
**2.** Composition cosmétique selon la revendication 1, caractérisée en ce que la phase à base de zinc du matériau composite est présente à la surface de la terre de diatomée.
**3.** Composition cosmétique selon la revendication 1 ou 2, caractérisée en ce que la terre de diatomée contient du carbonate de calcium.
**4.** Composition cosmétique selon la revendication 1 ou 2, caractérisée en ce que la terre de diatomée ne contient pas de carbonate de calcium.
**5.** Composition cosmétique selon l'une quelconque des revendications 1 à 4 pour son utilisation dans un traitement cosmétique.

Ainsi, la présente invention a pour but de fournir un matériau composite à base de terre de diatomée obtenu en faisant croître une phase cristalline spécifique à la surface du squelette de diatomée.

La présente invention a également pour but de fournir un matériau composite à base de terre de diatomée ayant les propriétés de la terre de diatomée combinées à celles de la phase cristalline nouvellement créée.

La présente invention concerne un matériau composite comprenant de la terre de diatomée et une phase à base de zinc.

Le terme "terre de diatomée" désigne l'ensemble formé par le squelette de diatomée, les phases annexes tels que le quartz et l'éventuel carbonate de calcium.

De préférence, la terre de diatomée utilisée est de la terre de diatomée brute (non calcinée), c'est-à-dire une terre de diatomée n'ayant pas subi de calcination au préalable. En effet, la calcination conduit à une condensation des groupements silanols sous forme de ponts siloxanes, ce qui limite la réactivité de surface du squelette de diatomée. Par ailleurs, la calcination conduit à l'oxydation du carbonate de calcium.

Selon un autre mode de réalisation, on peut utiliser de la terre de diatomée à laquelle on ajoute du carbonate de calcium.

La phase à base de zinc susmentionnée est une phase cristalline.

De préférence, la phase à base de zinc susmentionnée est présente à la surface de la terre de diatomée.

De façon encore plus préférée, ladite phase à base de zinc est présente uniquement à la surface de la terre de diatomée.

Selon un mode de réalisation préféré, la phase formée à base de zinc contient des carbonates, des chlorures, des nitrates, des sulfates, des phosphates, des hydroxyles, des oxydes, ou des mélanges de ceux-ci.

La présente invention concerne également un matériau composite tel que défini ci-dessus, caractérisé en ce que la terre de diatomée contient du carbonate de calcium.

De préférence, la terre de diatomée contient au moins 1% de carbonate de calcium, notamment de 15% à 60%, et de préférence de 15% à 40% de carbonate de calcium.

La présente invention concerne également un matériau composite tel que défini ci-dessus, caractérisé en ce que la terre de diatomée ne contient pas de carbonate de calcium.

Ainsi, la présente invention concerne notamment la croissance d'une phase cristalline à partir d'un sel de zinc à la surface d'une terre de diatomée grâce au carbonate de calcium la composant.

Selon un mode de réalisation avantageux, le matériau composite de l'invention est caractérisé en ce que l'organisation de la phase cristalline à base de zinc est de type lamellaire, sphérique ou sous forme d'aiguilles. Ladite phase à base de zinc peut toutefois également présenter une morphologie non spécifique.

Le matériau composite selon l'invention est également caractérisé en ce que la phase cristalline à base de zinc présente une morphologie de type rose des sables, sphérique ou sous forme d'aiguilles.

Les matériaux composites selon l'invention sont donc des composés mixtes terre de diatomée / zinc élaborés par cristallisation de zinc à partir de son sel à la surface du squelette de diatomée.

Les matériaux selon l'invention sont des composés siliciques macroporeux de type squelette de diatomée, modifiés comme mentionné ci-dessus, c'est-à-dire des matériaux de type squelette de diatomée comprenant une phase cristalline à base de zinc.

Les matériaux composites de l'invention sont caractérisés en ce qu'ils comprennent une matrice à base de terre de diatomée brute et une phase cristalline à base de zinc.

Selon un mode de réalisation particulier, le matériau composite de l'invention est caractérisé en ce que la terre de diatomée est choisie dans le groupe constitué des matériaux siliciques macroporeux d'algues eucaryotes fossilisées.

La présente invention décrit également un matériau composite tel que défini ci-dessus, nommé par la suite composite A, dans lequel la phase à base de zinc est une phase cristalline de type hydroxy carbonate de zinc.

La présente invention concerne un matériau composite tel que défini ci-dessus, nommé par la suite composite B, dans lequel la phase à base de zinc est une phase cristalline à base d'oxyde de zinc (II).

La présente invention décrit un procédé de préparation d'un matériau composite tel que défini ci-dessus, comprenant les étapes suivantes :
- une étape de mise en présence d'une solution aqueuse de terre de diatomée avec une solution aqueuse de zinc (II), et
- une étape de récupération dudit matériau composite, notamment par filtration ou centrifugation,
- une étape de séchage ou de lyophilisation.

Selon un mode de réalisation préféré, le procédé tel que défini ci-dessus est caractérisé en ce que la solution aqueuse de terre de diatomée présente une concentration en terre de diatomée comprise de 0,1 g.L⁻¹ à 100 g.L⁻¹, notamment de 1 g.L⁻¹ à 50 g.L⁻¹, et de préférence de 5 g.L⁻¹ à 20 g.L⁻¹.

Selon un mode de réalisation préféré, le procédé tel que défini ci-dessus est caractérisé en ce que la solution aqueuse de zinc (II) présente une concentration en zinc (II) comprise de 5.10⁻³ mol.L⁻¹ à 5.10⁻¹mol.L⁻¹, notamment de 5.10⁻² mol.L⁻¹ à 2.10⁻¹ mol.L⁻¹, et de préférence égale à 0,2 mol.L⁻¹.

Selon un mode de réalisation préféré, la solution aqueuse de zinc est sous la forme de chlorure, nitrate, sulfate, phosphate, carbonate ou hydroxyde.

De préférence, le procédé tel que mentionné ci-dessus comprend une étape subséquente de traitement thermique dudit matériau composite.

Ainsi, la présente invention décrit un procédé de préparation du matériau composite A tel que défini ci-dessus, comprenant les étapes suivantes :
- une étape de mise en présence d'une solution aqueuse de terre de diatomée avec une solution aqueuse de zinc (II),
- une étape d'agitation du mélange ainsi obtenu à une température comprise de 25°C à 150°C, de préférence à température ambiante, pendant une durée de 30 minutes à 72 heures, de préférence pendant 48 heures,
- une étape de récupération dudit matériau composite, notamment par filtration ou centrifugation, et
- une étape de séchage en étuve à une température comprise de 30°C à 80°C, de préférence à 60°C, pendant une durée de 12 heures à 36 heures, de préférence pendant 24 heures.

La présente invention décrit un procédé de préparation du matériau composite B tel que défini ci-dessus, comprenant une étape de traitement thermique du matériau composite A susmentionné, à une température comprise de 150°C à 900°C, de préférence à 700°C, pendant une durée de 30 minutes à 6 heures, de préférence pendant 2 heures.

Ainsi, le mode opératoire relatif à l'élaboration du composite terre de diatomée / oxyde de zinc (II) (composite B) se décompose en deux étapes. La première est la préparation d'un matériau intermédiaire (composite A), où la phase zinc (II) est dispersée sur la matrice hôte (frustule de diatomée). La deuxième étape concerne une étape de calcination du matériau intermédiaire, conduisant à l'obtention de la phase oxyde de zinc (II), elle-même dispersée à la surface des squelettes de diatomée.

La présente invention concerne une composition cosmétique comprenant un matériau composite tel que défini ci-dessus, le composite B, en association avec un véhicule cosmétiquement acceptable.

L'expression "véhicule cosmétiquement acceptable" désigne un véhicule adapté pour une utilisation en contact avec des cellules humaines et animales, en particulier les cellules de l'épiderme, sans toxicité, sans irritation, sans réponse allergique indue et similaire, et proportionné à un rapport avantage/risque raisonnable.

En particulier, le matériau composite utilisé selon la présente invention peut être mis en oeuvre au sein d'une composition cosmétique se présentant sous forme d'onguent, de crème, d'huile, de lait, de pommade, de poudre, de tampon imbibé, de solution, de gel, de spray, de lotion, de suspension, de savon.

Les compositions de l'invention peuvent être des compositions cosmétiques sous la forme d'émulsion huile-dans-eau ou eau-dans-huile, ou d'émulsion multiple, de micro-émulsion, de gel hydroalcoolique, de crème, d'huile, de lotion hydroalcoolique.

De préférence, la composition cosmétique selon l'invention est destinée à une application topique.

La présente invention concerne également une méthode de traitement cosmétique comprenant l'application d'une composition cosmétique telle que définie ci-dessus.

### FIGURES

Figure 1 : Diffractogramme de rayons X du composite A de l'exemple 1.
Figure 2 : Clichés par microscope électronique à balayage (MEB) du composite A de l'exemple 1.
Figure 3 : Diffractogramme de rayons X du composite B de l'exemple 2.
Figure 4 : Clichés par microscope électronique à balayage (MEB) du composite B de l'exemple 2.
Figure 5 : spectres enregistrés par spectroscopie UV visible en phase solide sur un domaine spectral s'étendant de 200 à 500 nm. Pourcentage d'absorbance (%) en fonction de la longueur d'onde (nm).

### EXEMPLES

### Exemple 1

### Préparation du composite intermédiaire à partir de terre de diatomée et d'une solution de chlorure de zinc (II) (composite A)

1 g de terre de diatomée (composition massique : 60% SiO₂ et 40% CaCO₃ ; surface spécifique (SBET) environ égale à 10 m².g-¹) est mis en suspension dans un bécher contenant 100 mL d'eau distillée sous agitation. Dans un deuxième bécher de 100 mL, une solution aqueuse 0,2 molaire de chlorure de zinc (II) est préparée. Après 10 minutes d'agitation, la solution de zinc (II) est ajoutée à la solution de terre de diatomée sous agitation à température ambiante pendant 48 heures. A l'issue de ce temps, le matériau est récupéré par filtration sur Büchner et mis à sécher en étuve pendant une durée de 24 heures à 60°C.

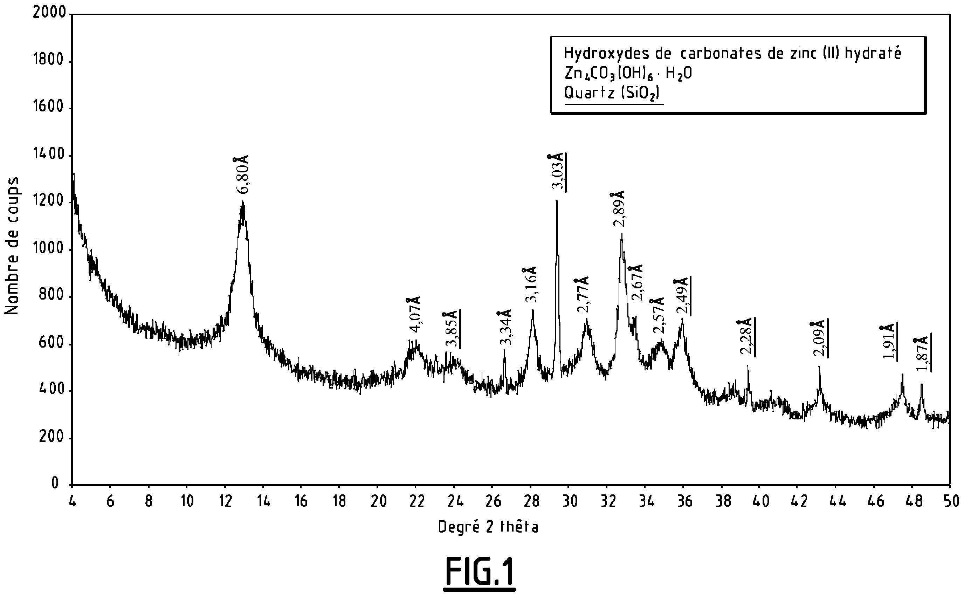

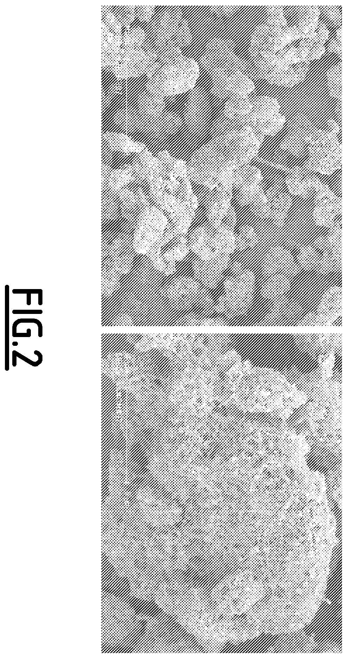

La diffraction de rayons X réalisée sur le matériau (composite A) fait apparaître une unique phase cristalline à base de zinc (II), identifiée comme un hydroxyle de carbonate de zinc (II) hydraté de formule chimique Zn₄CO₃(OH)₆ ·H₂O (Figure 1). L'analyse par fluorescence X opérée sur le matériau permet d'estimer la masse de zinc présent dans la composition chimique de la terre de diatomée, dans ce cas environ 27% en masse du composite. Les clichés de microscopie à balayage (Figure 2) montrent la présence de la phase à base de zinc à la surface de la terre de diatomée. Enfin, le composite ainsi obtenu présente une surface spécifique (S_{BET}) de l'ordre d'environ 40 m².g⁻¹.

### Exemple 2

### Préparation du composite à partir du composite A de l'exemple 1 (composite B)

1 g de terre de diatomée / Zn₄CO₃(OH)₆ ·H₂O (composite A) de composition massique des principaux composants, exprimée sous forme d'oxyde : 55% SiO₂, 35% ZnO, 10% C) est placé dans un creuset puis dans un four pour calcination. Le programme considéré consiste en une montée de 25 à 700°C pendant deux heures (i.e. une vitesse de montée de 5,83°C/minute), puis un pallier de 2 heures et un refroidissement à température ambiante.

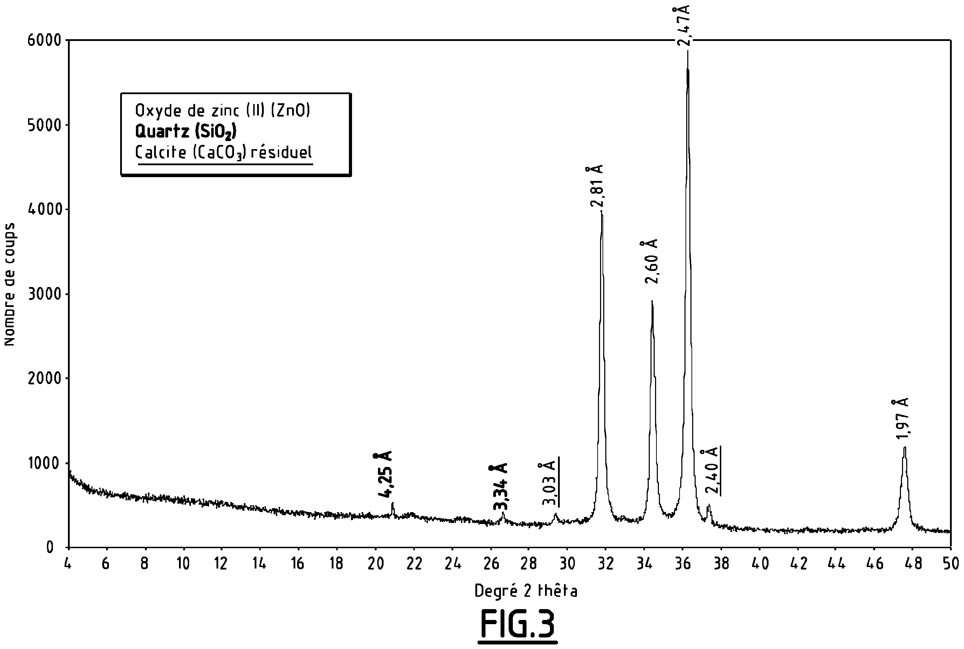

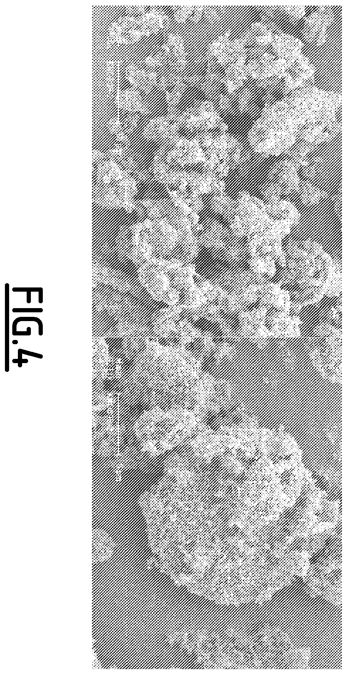

La diffraction de rayons X réalisée sur le matériau ainsi obtenu permet d'observer l'apparition d'une nouvelle phase cristalline de type oxyde de zinc (II) (Figure 3), de formule chimique ZnO. Les clichés de microscopie à balayage (Figure 4) montrent la présence de particules de ZnO de dimension de l'ordre de la centaine de nanomètres. Enfin, le composite ainsi obtenu présente une surface spécifique (S_{BET}) de l'ordre d'environ 15 m².g⁻¹.

### Exemple 3 - Tests d'absorption UV

Le composite B susmentionné (exemple 2) a été testé pour établir ses propriétés de rétention du rayonnement UV.

De même, pour comparaison, les mêmes propriétés ont été mesurées pour la terre de diatomée brute et pour l'oxyde de zinc (II) pur (à 98%).

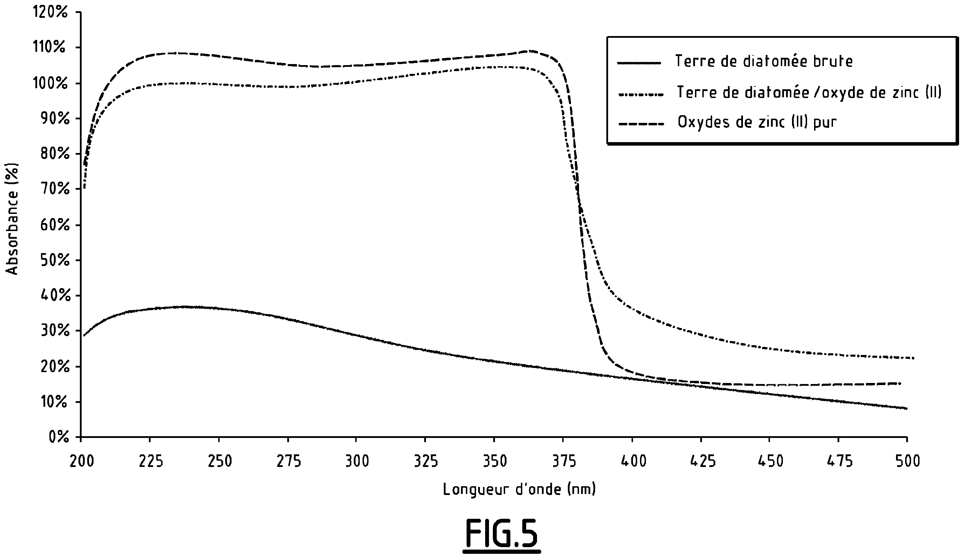

Ainsi, d'après la Figure 5, il peut être conclu que le composite terre de diatomée / oxyde de zinc (II) (ou composite B) possède des propriétés de rétention du rayonnement UV supérieures à celles de la terre de diatomée brute. Ces propriétés sont en outre relativement proches de celle de l'oxyde de zinc (II) conformément aux observations faites à partir du spectre.

Le composite étant constitué de 50 à 75% en masse de ZnO selon les quantités de réactifs employées, l'avantage du composite par rapport aux oxydes de zinc (II) réside entre autre, dans une capacité d'absorption du rayonnement UV équivalente avec une quantité de ZnO moins importante. Ces propriétés sont la résultante de la dispersion des particules d'oxydes de zinc (II) à la surface des squelettes de diatomée.

### Exemple 4 - Applications cosmétiques

### Principe

Une crème contenant le composite B susmentionné de l'exemple 2 à raison de 10% en poids a été préparée et son indice de protection ou facteur de protection solaire (FPS ou SPF pour "*sun protection factor*")*.*

Ce facteur a été déterminé selon la méthode internationale d'essai du facteur de protection solaire SPF ("*International Sun Protection Factor Test Method*")(Directive Européenne 2006/647/CE).

On rappelle ici que ce facteur de protection juge le pouvoir d'un produit contre les coups de soleil et concerne donc la protection anti-UVB.

### Résultats

### Volontaires :

Les tests ont été effectués sur 6 volontaires humains sains (âgés de 29 à 63 ans)(phototype I, Il ou III) sans problème de photosensibilité.

Les catégories de peaux sont les suivantes :
peau de type I : peau qui rougit toujours et ne bronze jamais
peau de type II : peau qui rougit fréquemment et bronze à peine
peau de type II : peau qui rougit occasionnellement et bronze modérément

### Source UV :

La source UV est une simulation de lampe solaire Xenon 300W (Modèle 601-300 Multiport, Solar Light Co. Inc., Philadelphie, USA) et est conforme à la Méthode Internationale d'essai du facteur de protection solaire (COLIPA, 2006).

### Méthode :

Comme mentionné ci-dessus, les tests de mesure sont effectués selon la Méthode Internationale d'essai du facteur de protection solaire (COLIPA, 2006).

### Résultats :

Le tableau ci-après donne les valeurs moyennes du facteur de protection solaire de la crème contenant le composite B pour 4 sujets valides ainsi que les valeurs moyennes du facteur de protection solaire d'une crème standard (P3 - COLIPA, 2006).

| Produit | SPF (moyenne) | SD |
|---|---|---|
| Crème selon l'invention | 13,1 | 2,4 |
| Crème standard P3 | 15,8 | 3,3 |

On constate donc que la valeur obtenue pour le facteur de protection solaire de la crème selon l'invention est dans la gamme souhaitée.

Par ailleurs, des essais complémentaires ont été effectués afin de comparer l'efficacité de la crème selon l'invention par rapport au produit commercial Zinclear^{®} également à base d'oxyde de zinc.

Ainsi, pour une crème à base de 10% d'oxyde de zinc, un facteur SPF de 10 a été obtenu, tandis que pour la crème selon l'invention à base de 10% de composite (comportant 9,3% d'oxyde de zinc), on a obtenu un facteur SPF de 13.

On constate donc que le produit de l'invention est plus efficace que le produit commercial avec un facteur de protection solaire supérieur de 30%.

## Revendications

1. Composition cosmétique comprenant un matériau composite en association avec un véhicule cosmétiquement acceptable, ledit matériau composite comprenant de la terre de diatomée et une phase à base de zinc, dans laquelle la phase à base de zinc du matériau composite est une phase cristalline à base d'oxyde de zinc (II).

2. Composition cosmétique selon la revendication 1, **caractérisée en ce que** la phase à base de zinc du matériau composite est présente à la surface de la terre de diatomée.

3. Composition cosmétique selon la revendication 1 ou 2, **caractérisée en ce que** la terre de diatomée contient du carbonate de calcium.

4. Composition cosmétique selon la revendication 1 ou 2, **caractérisée en ce que** la terre de diatomée ne contient pas de carbonate de calcium.

5. Composition cosmétique selon l'une quelconque des revendications 1 à 4 pour son utilisation dans un traitement cosmétique.

## Patentansprüche

1. Kosmetischen Zusammensetzung, umfassend ein Verbundmaterial in Verbindung mit einem kosmetisch annehmbaren Träger, wobei das Verbundmaterial Diatomeenerde und eine Phase auf der Grundlage von Zink umfasst, wobei die Phase auf der Grundalge von Zink des Verbundmaterials eine kristalline Phase auf der Grundlage von Zinkoxid (II) ist.

2. Kosmetische Zusammensetzung nach Anspruch 1, **dadurch gekennzeichnet, dass** die Phase auf der Grundlage von Zink des Verbundmaterials an der Oberfläche der Diatomeenerde vorhanden ist.

3. Kosmetische Zusammensetzung nach Anspruch 1 oder 2, **dadurch gekennzeichnet, dass** die Diatomeenerde Calciumcarbonat enthält.

4. Kosmetische Zusammensetzung nach Anspruch 1 oder 2, **dadurch gekennzeichnet, dass** die Diatomeenerde kein Calciumcarbonat enthält.

5. Kosmetische Zusammensetzung nach einem beliebigen der Ansprüche 1 bis 4 für ihre Verwendung bei einer kosmetischen Behandlung.

## Claims

1. A cosmetic composition comprising a composite material in association with a cosmetically acceptable vehicle, said composite material comprising diatomaceous earth and a zinc-based phase, wherein the zinc-based phase of the composite material is a crystalline phase of zinc (II) oxide.

2. The cosmetic composition according to claim 1, **characterized in that** the zinc-based phase of the composite material is present on the surface of the diatomaceous earth.

3. The cosmetic composition according to claim 1 or 2, **characterized in that** the diatomaceous earth contains calcium carbonate.

4. The cosmetic composition according to claim 1 or 2, **characterized in that** the diatomaceous earth does not contain calcium carbonate.

5. The cosmetic composition according to any of claims 1 to 4, for use thereof in a cosmetic treatment.
